# EUROPÄISCHE PATENTSCHRIFT

(11) **EP 2 340 809 B1**
(45) Veröffentlichungstag und Bekanntmachung des Hinweises auf die Patenterteilung: **15.04.2015**
(21) Anmeldenummer: 11151745.4
(22) Anmeldetag: 23.02.2007
(51) Int. Cl.: A61K 9/00, A61K 47/18

(54) **Arzneimittel enthaltend Fluorchinolone**
A medicament containing fluoroquinolones
Formulations de médicaments contenant des fluoroquinolones

(30) Priorität: 08.03.2006 DE 102006010643
(43) Veröffentlichungstag der Anmeldung: 06.07.2011
(62) Teilanmeldung aus: 07846476.5
(73) Patentinhaber: Bayer Intellectual Property GmbH, 40789 Monheim (DE)
(72) Erfinder: Dr. Heep, Iris, 50859 Köln (DE); Dr. Fraatz, Kristine, 51399 Burscheid (DE); Dr. Hamann, Hans-Jürgen, 41539 Dormagen (DE); Dr. Edingloh, Markus, 51379 Leverkusen (DE)
(74) Vertreter: BIP Patents

(56) Entgegenhaltungen:
- WO-A-00/64429
- WO-A-99/02130
- WO-A-99/29322
- WO-A-2004/082658
- WO-A-2005/018641

## Beschreibung

Die Erfindung betrifft die Verwendung von quartären Ammoniumverbindungen zur Verhinderung von Ausfällungen von Fluorchinolonen aus ihren Lösungen sowie stabile, verträgliche Arzneimittel, insbesondere geeignet parenteralen Anwendung, die in gelöster Form ein Fluorchinolon und eine quartäre Ammoniumverbindung enthalten.

Es gibt eine umfangreiche Forschung auf dem Gebiet der Solubilisierung von antibiotisch wirksamen Fluorchinolonen. So sind in DE-OS-3 831 514 Lösungen mit Chinolonen durch den Einsatz von Metallionen, insbesondere durch Calciumionen beschrieben. Eine verbesserte Löslichkeit von Chinolonen wird auch in JP 02-264724 sowie von M. Nakano, M. Yamamoto, T. Arita in Chem. Pharm. Bull., Interactions of Aluminium, Magnesium and Calcium ions with Nalidixic acid, 26 (5) 1505-1510 (1978) durch den Einsatz von Metallionen, insbesondere von Magnesiumionen beschrieben. In EP-A 507 851 sind Komplexe von Chinolonen mit Metallionen sowie Säure beschrieben; in diesen Komplexen soll die Löslichkeit der Chinolone verbessert sein. Durch Zusatz von Metallionen sowie Kosolventien ist die verbesserte Löslichkeit von einem Fluorchinolon in US 5811130 begründet. Die Verträglichkeit dieser Komplexe ist an Kälbern dargestellt. Komplexe von Chinolonen mit Metallionen und speziell mit Magnesiumionen sind auch in WO 99/29322 beschrieben, wodurch eine bessere Löslichkeit der Chinolone erreicht werden soll. Die hier beschriebenen Komplexe sind als gut verträglich und lagerstabil beschrieben, wobei die Verträglichkeit an Kälbern und Ratten untersucht wurde.

Lösungen für pharmazeutische Zwecke müssen frei von Ausfällungen sein. Bei Lösungen, die zur parenteralen Applikation bestimmt sind, ist dies besonders wichtig und auch Ausfällungen in geringem Maßstab, als Partikelbildung bezeichnet, sind nicht akzeptabel. Dies gilt für die gesamte Haltbarkeitsdauer eines pharmazeutisch genutzten Produktes. Es gibt daher viele Veröffentlichungen zu dem Thema der Vermeidung von Partikelbildung respektive Ausfällung von Arzneistoffen aus Lösungen. Z. B. ist in WO 98/18492 die Vermeidung von Partikelbildungen in Lösungen mit Cephalosporinen durch den Einsatz von Phospholipiden beschrieben.

WO 99/02130 offenbart eine stabile wässrige ophthalmische Zusammensetzung enthaltend 0,1 - 0,3 Gew.-% Ofloxacin, insbesondere Levofloxacin, oder das entsprechende Hydrochlorid, 0 - 1,0 Gew.-% Benzalkoniumchlorid sowie im übrigen Wasser.

WO0064429 offenbart Floxal® folgender Zusammensetzung: 1 ml enthaltend 3 mg Ofloxacin; weitere Bestandteile: 0.0025 Gew.-% Benzalkoniumchlorid und Wasser. Weiterhin offenbart dieses Dokument Siloxan® der Zusammensetzung: 1 ml enthaltend 3,5 mg Ciprofloxacin-HCl (3,5 mg = 3 mg Ciprofloxacin); 0,006 Gew.-% Benzalkoniumchlorid und Wasser.

In EP-A 287 926 ist speziell für die Synthese von Fluorchinolonen ein detailliertes Aufreinigungsverfahren beschrieben, damit es bei der Herstellung von (Injektions-) Lösungen nicht zur Partikelbildung kommt.

Ebenso ist in WO 01/10408 beschrieben, dass für die Herstellung von Injektionslösungen eines Fluorchinolons Einsatzstoffe mit einem besonders geringen Anteil an Verunreinigungen durch Metallionen verwendet werden müssen, damit gewährleistet ist, dass die Lösungen frei von Partikeln bleiben.

Ein ebenfalls häufig gewählter Ausweg aus der Problematik der Partikelbildung stellt z.B. die Gefriertrocknung von Lösungen dar.

Es ist allgemein bekannt, dass oberflächenaktive Substanzen bei parenteraler Gabe nicht gut verträglich sind. Dies ist unter anderem auf die Affinität der oberflächenaktiven Substanzen zu Bestandteilen der Zellwände zurückzuführen. Oberflächenaktive Substanzen mit einer besonders ausgeprägten Affinität zu Zellwänden werden deshalb u.a. auch als Desinfektionsmittel oder als Konservierungsmittel eingesetzt. Dies gilt auch für Konservierungsmittel aus der Gruppe der quartären Ammoniumverbindungen, wie z. B. Benzalkoniumchlorid.

Es ist ebenfalls allgemein bekannt, dass die Gruppe der quartären Ammoniumverbindungen hautreizend und schleimhautreizend sind. So beschreibt K.H. Wallhäuser (in Praxis der Sterilisation, Desinfektion-Konservierung, Thieme Verlag, 1995, 5. Auflage, S. 586-598) die hautreizende Wirkung von Benzalkoniumchlorid an der gesunden Kaninchenhaut; auch die Ausbildung von Dermatiden an Mäusen wird beschrieben (J. Amer. Vet. Med. Ass., 1972, 161 (6), 652-655. Dermatitis and death in mice accidentially exposed to quaternary ammonium disinfectants). In J. Gen. Microbiol., 1967, 48 (3), 391-400 ("Effects of organic cations on the gram-negative cell wall and their bactericidal activity with EDTA and surface active agents") ist der Mechanismus der Wirkung quartärer Ammoniumverbindungen dargelegt, wobei explizit die Wechselwirkung mit den Zellmembranen und die daraus resultierende Erhöhung der Permeabilität für andere Substanzen beschrieben ist.

Die Verbesserung der Löslichkeit von Fluorchinolonen durch den Zusatz von Metallionen wie z.B. von Magnesiumionen oder Calciumionen ist zwar im Prinzip in der Literatur beschrieben. Dennoch weisen in einigen Fällen Lösungen von Fluorchinolonen bei Lagerung die bekannten Ausfällungen in Form von Partikelbildungen auf. Dies geschieht auch unter Verwendung der allgemein üblichen Substanzen zur Vermeidung von Ausfällungen oder Kristallisationen. So wird z.B. durch Einsatz der in US 5 811 130 beschriebenen Kosolventien oder auch durch säurehaltige Formulierungen, wie in EP-A 507 851 beschrieben, eine Partikelbildung in Lösungen von Pradofloxacin nicht unterbunden.

Zudem sind Formulierungen der Fluorchinolone in den unterschiedlichen Tierspezies nicht gleichermaßen gut verträglich, so dass von einer Verträglichkeit, die an Kälbern ermittelt wurde, nicht auf eine Verträglichkeit an z.B. Schweinen oder Hunden oder gar Katzen ausgegangen werden kann. Dies gilt auch für den umgekehrten Fall; Injektionslösungen, die an Hunden verträglich sind, sind nicht unbedingt an Katzen oder Kälbern verträglich.

Im Unterschied zum Nutztierbereich (z. B. Rinder), ist neben den objektiven lokalen Befunden die Bewertung der lokalen Toleranz von Injektionslösungen beim Hobbytier (z. B. Hunde oder Katzen) kritischer. So ist die Akzeptanz der Tierbesitzer gegenüber leichten lokalen Unverträglichkeiten (z.B. Schwellungen, Schmerzhaftigkeit) im Hobbytierbereich deutlich niedriger als im Nutztierbereich. Darüber hinaus reagieren besonders die im Hobbytierbereich anzutreffenden Züchtungsvarianten oft empfindlicher auf subkutane Injektionen verglichen mit Nutztieren. Hier sind besonders die Katzen als sehr empfindliche Tierart zu nennen. Es ist daher auch nicht verwunderlich, dass die meisten Injektionslösungen mit Fluorchinolonen unter anderem aufgrund der mangelnden Verträglichkeiten nicht für Hunde oder Katzen zur Verfügung stehen.

Um die Verträglichkeit möglichst gut zu gestalten, empfiehlt es sich, den pH-Wert der Lösungen möglichst neutral (ca. 7,4) zu halten, was aber der Löslichkeit der Fluorchinolone widerspricht, da diese bei neutralem pH in der Regel eine besonders geringe Löslichkeit haben. Dies gilt insbesondere für die bei Chinolonen bevorzugt verwendeten wässrigen Systeme, wobei wegen der schlechten Löslichkeit der Chinolone bei pH-Werten um den Neutralpunkt auf Zusätze wie Kosolventien zurückgegriffen wird. Im neutralen pH-Bereich ist häufig eine Partikelbildung der Betainform der Fluorchinolone zu beobachten, weshalb Lösungen, wenn sie schon verträglich sein sollten, dann aber oft nicht lange lagerfähig sind und es zur Partikelbildung kommt. Dies wird in der Praxis zum Beispiel dadurch umgangen, dass man auf gefriergetrocknete Produkte ausweicht. Gefriergetrocknete Produkte sind aber in der Praxis umständlich zu handhaben und die rekonstituierte Lösung weist in der Regel eine kurze Haltbarkeit (z. B. 4 Wochen) nach Rekonstitution auf oder muss aufgrund der möglichen Partikelbildung direkt verworfen werden. Solche Produkte haben also gegenüber einer fertigen, direkt applizierbaren Injektionslösung klare Nachteile.

Von Vorteil ist demnach eine gebrauchsfertige Lösung als Injektionslösung, auch als "ready to use - Formulierung" bezeichnet. Des weiteren ist es erforderlich, dass nach Applikation, wie auch in WO 99/29322 beschrieben, das Fluorchinolon in der entsprechenden Menge in das Serum übergeht. Auch dies ist mit injizierbaren Formulierungen von Fluorchinolonen nicht selbstverständlich und kann ebenfalls von den jeweiligen Tierspezies abhängen.

Es wurde eine Möglichkeit gefunden Fluorchinolone mit pharmazeutisch annehmbaren Zusatzstoffen zuverlässig in Lösung zu halten. So können direkt anwendbare (ready to use) Formulierungen mit Fluorchinolonen bereitgestellt werden, die eine ausreichende Konzentration des Fluorchinolons enthalten, die nach parenteraler Applikation bei verschiedenen Tierspezies gut lokal verträglich sind, die unter pharmazeutischen Lagerbedingungen stabil, sowie frei von Partikelbildung sind, und die ein vorteilhaftes serumkinetisches Profil aufweisen.

Gefunden wurde die Verwendung von quartären Ammoniumverbindungen zur Verhinderung von Ausfällungen von Fluorchinolonen aus ihren Lösungen.

Die Erfindung betrifft:
Ein Arzneimittel enthaltend in gelöster Form:
   (a) (a) 1 bis 15 % (M/V) Pradofloxacin und
   (b) 0,001 bis 10 % einer quartären Ammoniumverbindung.

Fluorchinolone sind Verbindungen, wie sie unter anderem in folgenden Dokumenten offenbart sind: US 4 670 444 (Bayer AG), US 4 472 405 (Riker Labs), US 4 730 000 (Abbott), US 4 861 779 (Pfizer), US 4 382 892 (Daiichi), US 4 704 459 (Toyama), als konkrete Beispiele seien genannt: Benofloxacin, Binfloxacin, Cinoxacin, Ciprofloxacin, Danofloxacin, Difloxacin, Enoxacin, Enrofloxacin, Fleroxacin, Ibafloxacin, Levofloxacin, Lomefloxacin, Marbofloxacin, Moxifloxacin, Norfloxaein, Ofloxacin, Orbifloxacin, Pefloxaein, Temafloxacin, Tosufloxacin, Sarafloxacin, Sparfloxacin.

Als besonders bevorzugte Fluorchinolone seien die in WO 97/31001 beschriebenen Verbindungen genannt, insbesondere 8-Cyan-1-cyclopropyl-7-((1S,6S)-2,8-diazabicyclo[4.3.0]nonan-8-yl)-6-fluor-1,4-dihydro-4-oxo-3-chinolincarbonsäure (Pradofloxacin) mit der Formel

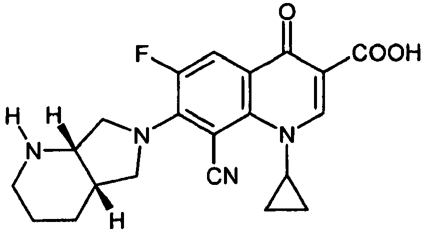

Die Fluorchinolone können in Form ihrer Racemate oder in enantiomeren Formen vorliegen. Sowohl die reinen Enantiomere als auch deren Mischungen können erfindungsgemäß eingesetzt werden.

Als Salze kommen pharmazeutisch verwendbare Säureadditionssalze und basische Salze in Frage.

Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen. Ferner lassen sich die erfindungsgemäßen Verbindungen an saure oder basische Ionenaustauscher binden. Als pharmazeutisch verwendbare basische Salze seien die Alkalisalze, beispielsweise die Natrium- oder Kaliumsalze, die Erdalkalisalze, beispielsweise die Magnesium-, oder Calciumsalze; die Zinksalze, die Silbersalze und die Guanidiniumsalze genannt.

Unter Hydraten werden sowohl die Hydrate der Fluorchinolone selbst als auch die Hydrate von deren Salzen verstanden. Als Beispiel sei Pradofloxacin genannt, das ein stabiles Trihydrat bildet (siehe WO 2005/097789).

Fluorchinolone können als Feststoff unter Umständen verschiedene Kristaltmodifikationen bilden. Vorteilhaft für die Arzneimittel der vorliegenden Erfindung sind solche Modifikationen, die entsprechende Löslichkeitseigenschaften haben.

In den erfindungsgemäßen Arzneimitteln wird das Fluorchinolon für Tiere mit einem Körpergewicht bis zu ca. 80 kg typischerweise in einem Anteil von 0,1 bis 15 %, bevorzugt 0,5 bis 15 % und besonders bevorzugt mit 1 bis 15 % eingesetzt. Bei Tieren mit einem Körpergewicht ab ca. 80 kg wird das Fluorchinolon typischerweise in einem Anteil für von 1 bis 30 %, bevorzugt 3 bis 25 % und besonders bevorzugt mit 4 bis 20 % eingesetzt. Hier und im Folgenden sind - soweit nicht anders angegeben - unter den Prozentangaben Prozent (M/V) zu verstehen. Dies bedeutet: Masse der betreffenden Substanz in Gramm pro 100 ml fertiger Lösung.

Die erfindungsgemäßen Arzneimittel können weitere geeignete Wirkstoffe wie z.B. Analgetika, insbesondere NSAID's (nichtsteroidale, antiinflammatorische Substanzen), enthalten. Solche NSAID's können z.B. sein: Meloxicam, Flunixin, Ketoprofen, Carprofen, Metamizol oder (Acetyl-) Salicylsäure.

Quartäre Ammoniumverbindungen im Sinne dieser Erfindung sind üblicherweise organische Ammoniumverbindungen, die unpolare Substituenten aufweisen und verschiedene Gegenionen wie z. B. Chlorid, Bromid, Iodid oder Fluorid haben können. Bevorzugt sind dies Verbindungen der allgemeinen Formel **(III):**

[R¹R²R³R⁴N]⁺ X⁻ (III)

wobei
- R¹ bis R⁴: gleich oder verschieden sind und für C₁₋₁₈-Alkyl stehen, das gegebenenfalls ein- oder mehrfach durch Sauerstoff unterbrochen sein kann und mit Hydroxyl oder mit einem gegebenenfalls mit einem oder mehreren Halogenatomen oder C₁₋₈-Alkylresten substituierten Arylrest substituiert sein kann, oder
- R¹ bis R⁴: durch Ringschluss von drei Resten 5- oder 6-gliedrige heterocyclische Reste, wie z. B. Pyridin oder Thiazolin, bilden, die ihrerseits gegebenenfalls ein oder mehrfach mit C₁₋₄-Alkyl oder C₁₋₄-Alkenyl substituiert sind, die gegebenenfalls einen Arylrest tragen, der seinerseits mit Halogen, inbesondere Chlor, Amino oder Dimethylamino substituiert sein kann, und
- X: für Sulfat, Halogenid, insbesondere Chlorid, Bromid oder Iodid, oder ein ähnliches Gegenion steht.

Mindestens einer der Reste R¹ bis R⁴ weist vorzugsweise eine Kettenlänge von 8 bis 18, besonders bevorzugt 12 bis 16 C-Atomen auf.

Aryl steht bevorzugt für einen Phenylrest, der gegebenenfalls mit 1 oder 2 Resten ausgewählt aus Halogen, inbesondere Chlor, und C₁₋₈-Alkyl substituiert ist.

Beispiele sind Alkyl-dimethyl-benzylammoniumchloride, insbesondere Benzalkoniumchlorid [(C₈₋₁₈)-Alkyl-dimethyl-benzyl-ammoniumchloridl oder n-(C₁₂-C₁₈)-Alkyl-benzyldimethylammoniumchlorid mit durchschnittlichen Molekulargewichten von ca. 380, Benzethoniumchlorid (Diisobutylphenoxyethoxyethyl-dimethylbenzyl-ammoniumchlorid), Dichlorbenryl-dimethyl-alkyl-ammoniumchlorid, Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid, Di-(C₈-C₁₈)-alkyl-dimethylammoniumchlorid wie z.B. Dioctyl-dimethyl-ammoniumchlorid oder Di-n-decyl-dimethyl-ammoniumchlorid, Cetylpyridiniumchlorid (1-Hexadecyl-pyridiniumchlorid) sowie Thiazoliniodid (3-Heptyl-2-(3-heptyl-4-methyl-4-thiazolin-2-yliden-methyl)-4-methyl-thiazoliniumjodid). Von diesen besonders bevorzugt sind Benzethoniumchlorid und Benzalkoniumchlorid.

Die quartären Ammoniumverbindungen werden üblicherweise in Konzentrationen von 0,001 bis 10 %, bevorzugt von 0,005 bis 6 % und besonders bevorzugt von 0,005 bis 3 % eingesetzt. Die Prozentangaben bedeuten % (M/V).

Die erfindungsgemäßen Arzneimittel können zusätzlich zu den quartären Ammoniumverbindungen weitere Substanzen enthalten, die eine Partikelbildung vermeiden können; z.B. Poloxamere, Lecithine, Polyvinylpyrrolidone, Kosolventien, Antioxidantien oder Komplexbildner. Auch diese werden üblicherweise in Konzentrationen von 0,001 bis 20 %, bevorzugt von 0,01 bis 10 % und besonders bevorzugt von 0,05 bis 3 % eingesetzt. Die Prozentangaben bedeuten % (M/V).

Die flüssigen Formulierungen können Substanzen enthalten, welche die lokale Verträglichkeit bei Applikation verbessern. Als Beispiele seien genannt: Radikalfänger oder Antioxidantien wie z.B. Vitamin E, wasserlösliche Vitamin E-Ester oder Vitamin C, Butylhydroxyanisol, Butylhydroxytoluol, Cysteamin, Cystein, Glutathion, Thioglykol, Thiomilchsäure, Natriumdisulfid oder auch Acetylcystein. Komplexbildner wie z.B. Cyclodextrine (z. B. Hydroxyproypl-Cyclodextrin), Natrium-EDTA (Ethylendiamintetraessigsäure), Polyvinlypyrrolidon, Dexpanthenol, Salze von Fettsäuren wie z.B. Natriumcaprylat, Salze mehrwertiger Metallkationen (z. B. Me²⁺ bzw. Me³⁺), insbesondere der Erdalkalimetalle und hier insbesondere das Magnesium in seinen Salzformen, Aminosäuren und hierunter besonders Arginin oder Lysin, Poloxamere, Poloxamine, Kosolventien wie z.B. n-Butanol, Glycerin, Polyethylenglykol, Propylenglykol oder Dimethylacetamid, Dextrane, Kreatin, Kreatinin, Säuren wie z.B. die Gluconolactonsäure, Milchsäure, Embonsäure, Zitronensäure, Weinsäure, Schleimsäure oder Hyaluronsäure, Lecithine mit einem Gehalt an Phosphatidylcholin von 70-100 % aus Soja oder Hühnereiweiß. Von den vorstehend genannten Substanzen werden bevorzugt die Salze mehrwertiger Metallkationen eingesetzt, und zwar bevorzugt die Erdalkalimetallsalze insbesondere Magnesiumsalze.

Substanzen, die die Verträglichkeit verbessern, sind üblicherweise in Konzentrationen von 0,05 bis 10 %, bevorzugt mit 0,1 bis 8 % und besonders bevorzugt mit 0,5 bis 5 % eingesetzt. Die Prozentangaben bedeuten % (M/V).

Als Solvens kann die flüssige Formulierung Wasser oder wassermischbare Substanzen enthalten. Als Beispiele seien genannt Glycerin, Propylenglykol, Polyethylenglykole, verträgliche Alkohole wie Ethanol, Benzylalkohol oder n-Butanol, Ethyllactat, Ethylacetat, Triacetin, N-Methylpyrrolidon, Propylencarbonat, Glycofurol, Dimethylacetamid, 2-Pyrrolidon, Isopropylidenglycerol, oder Glycerinformal. Kombinationen der Solventien sind ebenfalls denkbar. Bevorzugt sind Formulierungen auf Wasserbasis, in denen selbstverständlich weitere Solventien und Kosolventien enthalten sein können.

Als Solvens kann die flüssige Formulierung außer Wasser oder wassermischbaren Substanzen auch Öle in Form einer Emulsion enthalten. Hierunter seien die pflanzlichen, tierischen und synthetischen Öle wie Baumwollsamenöl, Sesamöl, Sojaöl, Mittelkettige Triglyceride einer Kettenlänge von C₁₂-C₁₈, Propylenglykoloctanoat-decanoat oder auch Paraffin genannt.

Das Solvenz ist üblicherweise in Konzentrationen von bis zu 98,5 %, bevorzugt bis zu 97% besonders bevorzugt bis zu 96,5% enthalten. In der Regel liegen die Konzentrationen des Solvenz über 50 %, bevorzugt über 60%, besonders bevorzugt über 70%. Die Prozentangaben bedeuten % (M/V).

Die erfindungsgemäßen Formulierungen können auch Kosolventien enthalten, und zwar bevorzugt dann wenn die Formulierungen Wasser enthalten; die Kosolventien können die Löslichkeit bestimmter Formulierungsbestandteile verbessern. Die Kosolventien werden üblicherweise in Anteilen von 1 bis 10 % bevorzugt von 3 bis 8 % eingesetzt (Prozentangaben jeweils M/V). Als Kosolventien seien beispielsweise genannt: Pharmazeutisch verträgliche Alkohole, Dimethylsulfoxid, Ethyllactat, Ethylacetat, Triacetin, N-Methylpyrrolidon, Propylencarbonat, Propylenglykol, Glycofurol, Dimethylacetamid, 2-Pyrrolidon, Isopropylidenglycerol, Glycerinformal, Glycerin und Polyethylenglykole. Als Kosolvenz eignen sich insbesondere pharmazeutisch verträgliche Alkohole, wie z. B. Ethanol, Benzylalkohol oder n-Butanol. Auch Mischungen der vorstehend genannten Lösungsmittel können als Kosolvenz eingesetzt werden.

Konservierungsmittel können in der flüssigen Formulierung enthalten sein. Die oben genannten quartären Ammoniumverbindungen haben in der Regel konservierende Wirkung, z. B. Benzalkoniumchlorid, Benzethoniumchlorid oder Cetylpyridiniumchlorid. Als Beispiele für weitere verwendbare Konservierungsmittel seien genannt: Aliphatische Alkohole wie Benzylalkohol, Ethanol, n-Butanol, Phenol, Cresole, Chlorbutanol, para-Hydroxybenzoesäureester (insbesondere die Methyl- und Propylester), Salze oder die freien Säuren der Carbonsäuren, wie Sorbinsäure, Benzoesäure, Milchsäure oder Propionsäure.

Je nach Art der Formulierung und Applikationsform können die erfindungsgemäßen Arzneimittel weitere übliche, pharmazeutisch verträgliche Zusatz- und Hilfsstoffe enthalten. Als Beispiele seien genannt
- Antioxidantien wie zum Beispiel Sulfite (Na-sulfit, Na-metabisulfit), organische Sulfide (Cystin, Cystein, Cysteamin, Methionin, Thioglycerol, Thioglykolsäure, Thiomilchsäure) Phenole (Tocopherole, wie auch Vitamin E und Vitamin-E-TPGS (d-alpha-Tocopherylpolyethylenglykol-1000-succinat)), Butylhydroxyanisol, Butylhydroxytoluol, Octyl- und Dodecylgallat), organische Säuren (Ascorbinsäure, Citronensäure, Weinsäure, Milchsäure) und deren Salze und Ester
- Netzmittel wie zum Beispiel Fettsäuresalze, Fettalkylsulfate, Fettalkylsulfonate, lineare Alkylbenzolsulfonate, Fettalkylpolyethylen-glykolethersulfate, Fettalkylpolyethylenglykolether, Alkylphenolpolyethylenglykolether, Alkylpolyglykoside, Fettsäure-N-methylglucamide, Polysorbate, Sorbitanfettsäureester und Poloxamere.
- Substanzen zur Isotonisierung wie z.B. Natriumchlorid, Glucose oder Glycerin.
- Pharmazeutisch akzeptable Farbstoffe wie zum Beispiel Eisenoxide, Carotinoide, etc.

Der pH-Wert der flüssigen Formulierungen beträgt 2-11, bevorzugt 3-8 und besonders bevorzugt 4-7,6.

Die erfindungsgemäßen Arzneimittel können hergestellt werden, indem das Fluorchinolon in dem Solvens dispergiert wird, die Substanzen zur Verbesserung der Verträglichkeit und gegebenenfalls zur Vermeidung von Partikelbildung ebenfalls ergänzt werden. Kosolventien sowie weitere Inhaltsstoffe wie z.B. Konservierungsstoffe können bereits dem Solvens zugegeben oder später zugemischt werden.

Alternativ können Kosolventien, Konservierungsmittel, Substanzen die die Verträglichkeit oder Partikelbildung beeinflussen auch zunächst im Solvens gelöst werden und anschließend erst das Fluorchinolon ergänzt werden.

Die erfindungsgemäßen pharmazeutischen Zubereitungen eignen sich generell für die Anwendung bei Mensch und Tier. Bevorzugt werden sie in der Tierhaltung und Tierzucht bei Nutz-, Zucht-, Zoo-, Labor-, Versuchs- und Hobbytieren eingesetzt.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär sowie Vögel wie z.B. Wachteln, Hühner, Gänse, Puten, Enten, Tauben und Vogelarten für Heim- und Zoohaltung.

Zu Labor- und Versuchstieren gehören Mäuse, Ratten, Meerschweinchen, Goldhamster, Kaninchen, Affen, Hunde und Katzen.

Zu den Hobbytieren gehören Kaninchen, Hamster, Ratten, Meerschweinchen, Mäuse, Pferde, Reptilien, entsprechende Vogelarten, Hunde und Katzen.

Weiterhin seien Fische genannt, und zwar Nutz-, Zucht-, Aquarien- und Zierfische aller Altersstufen, die in Süß- und Salzwasser leben.

Bevorzugt werden die erfindungsgemäßen Zubereitungen bei Hobbytieren wie Pferden, Kaninchen, Katzen und Hunden, eingesetzt. Insbesondere eignen sie sich für die Anwendung bei Katzen und Hunden.

Beispiele für bevorzugte Nutztiere sind Rind, Schaf, Schwein, Ziege, Pute und Huhn. Besonders bevorzugte Nutztiere sind Rind und Schwein.

Die Anwendung kann sowohl prophylaktisch, metaphylaktisch als auch therapeutisch erfolgen.

Bevorzugt werden die erfindungsgemäßen flüssigen Formulierungen als Lösungen oder Emulsion gegeben, besonders bevorzugt sind homogene Lösungen.

Die hier beschriebenen Formulierungen können auf verschiedene Arten dem Zielorganismus (Mensch oder Tier) zugeführt werden. Sie können beispielsweise parenteral, insbesondere durch Injektion (z. B. subkutan, intramuskulär, intravenös, intramammär, intraperitoneal), dermal, oral, rectal, vaginal oder nasal appliziert werden, wobei die parenterale Applikation - insbesondere durch Injektion - bevorzugt ist.

Die Verwendung mit den genannten Substanzen führt zu Arzneimitteln mit guter Löslichkeit des Wirkstoffs und guter Stabilität der Formulierung, insbesondere bezüglich Ausfällungen. Weiterhin zeichnet sich das erfindungsgemäße Arzneimittel durch gute Verträglichkeit und geeignete Serumkinetik bei den genannten verschiedenen Tierspezies insbesondere nach parenteraler Gabe aus.

### Beispiele

Die Formulierungen der folgenden Beispiele werden hergestellt, indem die angegebenen Einsatzstoffe in Aqua pro Injektione gemischt oder gelöst werden. Der pH-Wert der Lösungen kann durch Zugabe von Säuren oder Basen eingestellt werden. Die Lösungen zur Injektion werden sterilfiltriert und in geeignete Behältnisse überführt. Pradofloxacin kann als Anhydrat oder als Trihydrat eingesetzt werden; die Zahlenwerte sind jeweils für das Anhydrat berechnet.

(Prozentangaben in Gewichtsprozent bezogen auf das Gesamtvolumen des fertigen Präparats, [M/V]).

### Beispiel 1

1 % Enrofloxacin
3,0 % Magnesiumchloridhexahydrat
0,02 % Benzalkoniumchlorid
quantum satis Kaliumhydroxid
ad 100 % Aqua pro Injektione

0,5 g Enrofloxacin, 1,5 g Magnesiumchloridhexahydrat und 0,01 g Benzalkoniumchlorid werden in Aqua pro Injektione zu 50 ml gelöst und gegebenenfalls der pH-Wert mit Kaliumhydroxid auf 6,0 eingestellt.

### Beispiel 2

3,0 % Pradofloxacin (Trihydrat)
3,0 % Magnesiumchloridhexahydrat
0,02 % Benzalkoniumchlorid
quantum satis Natriumhydroxid
ad 100 % Aqua pro Injektione

1,5 g Pradofloxacin (als reines Pradofloxacin berechnet, als Trihydrat eingesetzt), 1,5 g Magnesiumchloridhexahydrat und 0,01 g Benzalkoniumchlorid werden in Aqua pro Injektione zu 50 ml gelöst und gegebenenfalls der pH-Wert mit Natriumhydroxid auf 6,0 eingestellt.

### Beispiel 3

1,5 % Pradofloxacin
3 % Magnesiumchloridhexahydrat
0,01 % Benzethoniumchlorid
ad 100 % Aqua pro Injektione

0,75 g Pradofloxacin, 1,5 g Magnesiumchloridhexahydrat und 0,005 g Benzethoniumchlorid werden in Aqua pro Injektione zu 50 ml gelöst und gegebenenfalls der pH-Wert mit Natriumhydroxid auf 6,0 eingestellt.

### Beispiel 4

1,5 % Pradofloxacin (Trihydrat)
3 % Magnesiumchloridhexahydrat
0,02 % Benzalkoniumchlorid
ad 100 % Aqua pro Injektione

0,75 g Pradofloxacin (als reines Pradofloxacin berechnet, als Trihydrat eingesetzt), 1,5 g Magnesiumchloridhexahydrat und 0,01 g Benzalkoniumchlorid werden in Aqua pro Injektione zu 50 ml gelöst und gegebenenfalls der pH-Wert mit Natriumhydroxid auf 6,0 eingestellt.

### Beispiel 5

5 % Pradofloxacin (Trihydrat)
0,02 % Benzalkoniumchlorid
3 % Magnesiumchloridhexahydrat
ad 100 % Aqua pro Injektione

80 g Aqua pro Injektione werden mit 0,02 g Benzalkoniumchlorid und 3 g Magnesiumchloridhexahydrat gemischt. Darin werden 5 g Pradofloxacin (als reines Pradofloxacin berechnet; als Trihydrat eingesetzt) gelöst. Mit den restlichen Aqua pro Injektione wird auf das Endgewicht zu 100 ml und zuvor gegebenenfalls der pH-Wert mit Natriumhydroxid auf 6,0 eingestellt.

### Beispiel 6

1,5 % Pradofloxacin (Trihydrat)
0,015 % Benzalkoniumchlorid
3 % Magnesiumchloridhexahydrat
ad 100 % Aqua pro Injektione

80 g Aqua pro Injektione werden mit 0,015 g Benzalkoniumchlorid und 3 g Magnesiumchloridhexahydrat gemischt. Darin werden 1,5 g Pradofloxacin (als reines Pradofloxacin berechnet; als Trihydrat eingesetzt) gelöst. Mit dem restlichen Aqua pro Injektione wird auf das Endgewicht zu 100 ml aufgefüllt und zuvor gegebenenfalls der pH-Wert mit Natriumhydroxid auf 6,0 eingestellt.

### Beispiel 7

1,5 % Pradofloxacin (Trihydrat)
0,01 % Benzalkoniumchlorid
3 % Magnesiumchloridhexahydrat
ad 100 % Aqua pro Injektione

80 g Aqua pro Injektione werden mit 0,01 g Benzalkoniumchlorid und 3 g Magnesiumchloridhexahydrat gemischt. Darin werden 1,5 g Pradofloxacin (als reines Pradofloxacin berechnet; als Trihydrat eingesetzt) gelöst. Mit dem restlichen Aqua pro Injektione wird auf das Endgewicht zu 100 ml aufgefüllt und zuvor gegebenenfalls der pH-Wert mit Natriumhydroxid auf 6,0 eingestellt.

### Verträglichkeit in vivo

Die hier beschriebenen Formulierungen haben im klinischen Versuch eine verbesserte lokale Verträglichkeit gegenüber anderen Formulierungen gezeigt. Wirkstoffabhängige Gewebeirritationen und Schwellungen an der Injektionsstelle sind in ihrer Ausprägung von der eingesetzten Formulierung abhängig. In der nachfolgenden Tabelle sind ausgewählte Beispiele dazu aufgelistet.

Das angewendete Testsystem besteht aus einer kombinierten Prüfung von lokaler Verträglichkeit initial und über 36 Tage und Serumpharmakokinetik von 0 - 72 h. Jede Formulierung wird an je 6 Tieren der beiden Spezies Hund und Katze nach subkutaner Einfachinjektion getestet. Die Serumproben (n = 11/Tier) werden mittels HPLC auf ihre Substanzkonzentration hin geprüft und die pharmakokinetischen Parameter daraus berechnet. Die lokale Verträglichkeit wird anhand der Parameter Schwellung, Schmerzhaftigkeit, Rötung und Hautirritation/-veränderung visuell und palpatorisch beurteilt.

**Tabelle 1. Ausgewählte Ergebnisse der klinischen Prüfung auf lokale Verträglichkeit verschiedener Formulierungen**

| | | Lokale Reaktionen (n) | | | | | | |
|---|---|---|---|---|---|---|---|---|
| Formulierung | (n) | Sofort | Tag 1 | Tag 7 | Tag 14 | Tag 21 | Tag 28 | Tag 36 |

| Katze: | | | | | | | | |
|---|---|---|---|---|---|---|---|---|
| Bsp. 4 | 6 | 0 | 0 | 0 | 0 | 0 | 0 | 0 |

| Hund: | | | | | | | | |
|---|---|---|---|---|---|---|---|---|
| Bsp. 2 | 9 | 0 | 0 | 0 | 0 | 0 | 0 | 0 |

| | | | | | | | | |
|---|---|---|---|---|---|---|---|---|
| (n): Anzahl der Tiere | | | | | | | | |

### Serumpharmakokinetisches Profil

Neben der unterschiedlichen Verträglichkeit, hat die Formulierung auch einen Einfluß auf das serumpharmakokinetische (PK) Profil. Verschiedene Formulierungen weisen deutliche Unterschiede in der Serumkonzentrations-Zeitkurve auf. Bevorzugt werden für Chinolone Kurven mit schneller Absorption, hohen Peakkonzentrationen und langen Eliminationsphasen. Die nachstehende Tabelle zeigt das PK-Profil einer erfindungsgemäßen Formulierung. Das verwendete Testsystem ist im Abschnitt "lokale Verträglichkeit" beschrieben worden.

**Tabelle 2. Ergebnisse zur Serumpharmakokinetik**

| | | PK Parameter (9 mg/kg, SC, Hund) | | | | |
|---|---|---|---|---|---|---|
| Formulierung | n | Cₘₐₓ (µg/mL) | Tₘₐₓ (hr) | t_{1/2} (hr) | AUC_{inf} (h*µg/mL) | AUCₗₐₛₜ (h*µg/mL) |
| Bsp.2 | 6 | 3.8 | 1.5 | 4.6 | 31.0 | 30.7 |

## Patentansprüche

1. Arzneimittel enthaltend in gelöster Form:
(a) 1 bis 15 % (M/V) Pradofloxacin und
(b) 0,001 bis 10 % einer quartären Ammoniumverbindung.

2. Arzneimittel gemäß Anspruch 1 enthaltend zwei- oder dreiwertige Metall-Kationen.

3. Arzneimittel gemäß Anspruch 2 enthaltend Mg²⁺.

4. Arzneimittel gemäß einem der Ansprüche 1 bis 3 enthaltend als quartäre Ammoniumverbindung eine Verbindung der Formel (III)
[R¹R²R³R⁴N]⁺ X⁻ (III)
wobei
R¹ bis R⁴ gleich oder verschieden sind und für C₁₋₁₈-Alkyl stehen, das gegebenenfalls ein-oder mehrfach durch Sauerstoff unterbrochen sein kann und mit Hydroxyl oder mit einem gegebenenfalls mit einem oder mehreren Halogenatomen oder C₁₋₈-Alkylresten substituierten Arylrest substituiert sein kann, oder
R¹ bis R⁴ durch Ringschluss von drei Resten 5- oder 6-gliedrige heterocyclische Reste, wie z. B. Pyridin oder Thiazolin, bilden, die ihrerseits gegebenenfalls ein oder mehrfach mit C₁₋₄-Alkyl oder C₁₋₄-Alkenyl substituiert sind, die gegebenenfalls einen Arylrest tragen, der seinerseits mit Halogen, inbesondere Chlor, Amino oder Dimethylamino substituiert sein kann, und
X für Sulfat, Halogenid, insbesondere Chlorid, Bromid oder Iodid, oder ein ähnliches Gegenion steht.

5. Arzneimittel gemäß einem der Ansprüche 1 bis 4 enthaltend als quartäre Ammoniumverbindung eine Verbindung ausgewählt aus: Alkyl-dimethyl-benzylammoniumchloride, insbesondere Benzalkoniumchlorid [(C₈₋₁₈)-Alkyl-dimethyl-benzyl-ammoniumchlorid] oder n-(C₁₂-C₁₈)-Alkyl-benzyldimethylammoniumchlorid mit durchschnittlichen Molekulargewichten von ca. 380, Benzethoniumchlorid (Diisobutylphenoxyethoxyethyl-dimethylbenzyl-ammoniumchlorid), Dichlorbenzyl-dimethyl-alkyl-ammoniumchlorid, Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid, Di-(C₈-C₁₈)-alkyl-dimethyl-ammoniumchlorid wie z.B. Dioctyl-dimethyl-ammoniumchlorid oder Di-n-decyl-dimethyl-ammoniumchlorid, Cetylpyridiniumchlorid (1-Hexadecyl-pyridiniumchlorid) sowie Thiazoliniodid (3-Heptyl-2-(3-heptyl-4-methyl-4-thiazolin-2-yliden-methyl)-4-methyl-thiazoliniumjodid).

6. Arzneimittel gemäß einem der Ansprüche 1 bis 5, zusätzlich enthaltend ein Analgetikum, insbesondere ein NSAID.

## Claims

1. Pharmaceutical containing, in dissolved form:
(a) from 1 to 15% (w/v) of pradofloxacin and
(b) From 0.001 to 10% of a quaternary ammonium compound.

2. Pharmaceutical according to Claim 1, containing di- or trivalent metal cations.

3. Pharmaceutical according to Claim 2, containing Mg²⁺.

4. Pharmaceutical according to one of Claims 1 to 3, containing, as quaternary ammonium compound, a compound of the formula (III)
[R¹R²R³R⁴N]⁺ X⁻ (III)
where
R¹ to R⁴ are identical or different and represent C₁₋₁₈-alkyl which can optionally be interrupted once or more than once by oxygen and which can optionally be substituted by hydroxyl or by an aryl radical which is optionally substituted by one or more halogen atoms or C₁₋₈-alkyl radicals, or
R¹ to R⁴ may, as the result of the cyclization of three radicals, form 5- or 6-membered heterocyclic radicals such as, for example, pyridine or thiazolin, which, in turn, are optionally mono- or polysubstituted by C₁₋₄-alkyl or C₁₋₄-alkenyl, which optionally have attached to them an aryl radical which, in turn, can be substituted by halogen, in particular chlorine, amino or dimethylamino, and
X represents sulphate, halide, in particular chloride, bromide or iodide, or a similar counterion.

5. Pharmaceutical according to one of Claims 1 to 4, containing, as quaternary ammonium compound, a compound selected among: alkyldimethylbenzylammonium chlorides, in particular benzalkonium chloride [(C₈₋₁₈)-alkyldimethylbenzylammonium chloride] or n-(C₁₂-C₁₈)-alkylbenzyldimethylammonium chloride with average molecular weights of approximately 380, benzethonium chloride (diisobutylphenoxyethoxyethyldimethylbenzylammonium chloride), dichlorobenzyldimethylalkylammonium chloride, benzoxonium chloride (benzyldodecylbis(2-hydroxyethyl)ammonium chloride), cetrimonium bromide (N-hexadecyl-N,N-trimethylammonium bromide, di-(C₈-C₁₈)-alkyldimethylammonium chloride such as, for example, dioctyldimethylammonium chloride or di-n-decyldimethylammonium chloride, cetylpyridinium chloride (1-hexadecylpyridinium chloride) and thiazoline iodide (3-heptyl-2-(3-heptyl-4-methyl-4-thiazolin-2-ylidenemethyl)-4-methylthiazolinium iodide).

6. Pharmaceutical according to one of Claims 1 to 5, additionally containing an analgesic, in particular an NSAID.

## Revendications

1. Médicament contenant sous forme dissoute :
(a) 1 à 15 % (m/v) de pradofloxacine et
(b) 0,001 à 10 % d'un composé d'ammonium quaternaire.

2. Médicament selon la revendication 1, contenant des cations métalliques bi- ou trivalents.

3. Médicament selon la revendication 2, contenant Mg²⁺ .

4. Médicament selon l'une quelconque des revendications 1 à 3, contenant en tant que composé d'ammonium quaternaire un composé de formule (III)
[R¹R²R³R⁴N] ⁺ X⁻ (III)
dans laquelle
R¹ à R⁴ sont identiques ou différents et représentent alkyle en C₁₋₁₈, qui peut éventuellement être interrompu une ou plusieurs fois par oxygène ou qui peut être substitué avec hydroxyle ou avec un radical acyle éventuellement substitué avec un ou plusieurs atomes d'halogène ou radicaux alkyle en C₁₋₈, ou
R¹ à R⁴ forment par fermeture de cycle de trois radicaux des radicaux hétérocycliques à 5 ou 6 éléments, tels que p. ex. pyridine ou thiazoline, qui sont de leur côté éventuellement substitués une ou plusieurs fois avec alkyle en C₁₋₄ ou alcényle en C₁₋₄, qui portent éventuellement un radical aryle, qui peut de son côté être substitué avec halogène, notamment chlore, amino ou diméthylamino, et
X représente sulfate, halogénure, notamment chlorure, bromure ou iodure, ou un contre-ion semblable.

5. Médicament selon l'une quelconque des revendications 1 à 4, contenant en tant que composé d'ammonium quaternaire un composé choisi parmi : le chlorure d'alkyl-diméthyl-benzylammonium, notamment le chlorure de benzalkonium [chlorure d'alkyle en (C₈₋₁₈)-diméthyl-benzyl-ammonium] ou le chlorure de n-alkyle en (C₁₂-C₁₈)-benzyldiméthylammonium ayant des poids moléculaires moyens d'environ 380, le chlorure de benzéthonium (chlorure de diisobutylphénoxyéthoxyéthyldiméthylbenzyl-ammonium), le chlorure de dichlorobenzyl-diméthyl-alkyl-ammonium, le chlorure de benzoxonium (chlorure de benzyl-dodécyl-bis-(2-hydroxyéthyl)-ammonium), le bromure de cétrimonium (bromure de N-hexadécyl-N,N-triméthyl-ammonium), le chlorure de di-alkyle en (C₈-C₁₈) -diméthyl-ammonium tel que p. ex. le chlorure de dioctyl-diméthyl-ammonium ou le chlorure de di-n-décyl-diméthyl-ammonium, le chlorure de cétyl-pyridinium (chlorure de 1-hexadécylpyridinium), ainsi que l'iodure de thiazoline (iodure de 3-heptyl-2-(3-heptyl-4-méthyl-4-thiazolin-2-ylidèneméthyl)-4-méthyl-thiazolinium).

6. Médicament selon l'une quelconque des revendications 1 à 5, contenant en outre un analgésique, notamment un AINS.
